# EUROPÄISCHE PATENTSCHRIFT

(11) **EP 2 162 652 B1**
(45) Veröffentlichungstag und Bekanntmachung des Hinweises auf die Patenterteilung: **11.09.2013**
(21) Anmeldenummer: 08774577.4
(22) Anmeldetag: 01.07.2008
(51) Int. Cl.: F16K 1/52, B05B 1/30, A61L 2/26, A61L 2/20

(54) **DOSIERVENTIL**
DOSING VALVE
SOUPAPE DOSEUSE

(30) Priorität: 10.07.2007 DE 102007031962
(43) Veröffentlichungstag der Anmeldung: 17.03.2010
(73) Patentinhaber: Krones AG, 93073 Neutraubling (DE)
(72) Erfinder: BURGMEIER, Berthold, 89561 Dischingen/Eglingen (DE); KNOTT, Josef, 84069 Schierling (DE)
(74) Vertreter: Bittner, Bernhard
(86) Internationale Anmeldenummer: PCT/EP2008/058430
(87) Internationale Veröffentlichungsnummer: WO 2009/007271

(56) Entgegenhaltungen:
- WO-A-89/04399
- US-A- 4 129 284
- US-A- 5 186 393

## Beschreibung

Die vorliegende Erfindung bezieht sich auf ein Dosierventil, und insbesondere ein Dosierventil, das in einem Verdampfer zum Sterilisieren von Behältnissen und speziell von Kunststoffbehältnissen Einsatz findet. Derartige Verdampfer sind aus dem Stand der Technik bekannt. Dabei wird üblicherweise Wasserstoffperoxid (H₂O₂) innerhalb des Verdampfers verdampft und gemeinsam mit einem zugeführten Luftstrom in ein zu sterilisierendes Behältnis geleitet. Durch dieses Einrühren des Gemisches in das Behältnis kann dessen Innenwandung gereinigt bzw. sterilisiert werden. Aus der WO 2007/03313 sind ein Verfahren und eine Vorrichtung zum Überwachen eines Verdampfers bekannt. Diese Vorrichtung weist dabei eine Einspritzdüse auf, deren Öffnung senkrecht nach unten auf die Mitte eines Heizkörpers gerichtet ist.

Bei derartigen Verdampfern ist es erforderlich, die Menge des jeweils eingespritzten oder dem Behältnis zuzuführenden H₂O₂ präzise zu regulieren und dafür Sorge zu tragen, dass pro Behältnis jeweils die gleiche Menge an H₂O₂ zugeführt wird.

Im Stand der Technik werden zum Einspritzein des Wasserstoffperoxids Zuleitungen und auch Ventilkörper eingesetzt. Diese Ventile arbeiten zwar zufriedenstellend, erlaubten jedoch nicht in jedem Fall eine hinreichend genaue Dosierung des einzuspritzenden Volumens.

Die WO 89/04399 beschreibt ein Ventil zum Sprühen von Teer. Dieses Ventil weist einen Kolben zum Verschließen einer Austrittsöffnung auf. Der heiße Teer fließ dabei unter Druck in einer Hohlstange.

Die US-A-4129284 zeigt die Merkmale des Oberbegriffs von Anspruch 1.

Der vorliegenden Erfindung liegt daher die Aufgabe zugrunde, ein Dosierventil zur Verfügung zu stellen, welches eine genaue Dosierung der in den Verdampfer einzuspritzenden Menge an Wasserstoffperoxid erlaubt. Dies wird durch den Gegenstand von Anspruch 1 erreicht. Vorteilhafte Ausführungsformen und Weiterbildungen sind Gegenstand der Unteransprüche.

Ein erfindungsgemäßes Dosierventil, insbesondere zum Dosieren einer Wasserstoffperoxid enthaltenden Substanz, weist ein Gehäuse auf sowie eine Zuleitung, um dem Dosierventil die Substanz zuzuführen. Weiterhin ist ein innerhalb des Gehäuses entlang einer geraden Richtung zwischen wenigstens zwei Stellungen bewegbarer Ventilkörper vorgesehen sowie eine in dem Dosierventil angeordnete Austrittsöffnung für die Substanz. Erfindungsgemäß weist diese Austrittsöffnung einen vorgegebenen Strömungsquerschnitt auf und dieser Strömungsquerschnitt der Austrittsöffnung bildet in einer geöffneten Stellung des Dosierventils den geringsten Strömungsquerschnitt des Dosierventils auf dem Pfad der Substanz durch das Dosierventil.

Bei den beiden Stellungen, zwischen denen der Verschlusskörper bewegbar ist, handelt es sich bevorzugt um eine geöffnete und eine geschlossene Stellung des Dosierventils. Unter dem Pfad der Substanz des Dosierventils wird der Weg verstanden, den die Substanz ausgehend von der Zuleitung bis hin zu der Austrittsöffnung einnimmt. Dadurch, dass die Austrittsöffnung selbst den geringsten Öffnungsquerschnitt bildet, wird erreicht, dass bei geöffnetem Zustand des Ventils die genaue Dosierung der in den Verdampfer zugeführten Substanz möglich ist, da die gesamte Durchflußmenge im Wesentlichen durch den Bereich mit dem engsten Strömungsquerschnitt und damit durch den Querschnitt bzw. auch den Durchmesser der Austrittsöffnung bestimmt wird. Wäre der Bereich mit dem geringsten Strömungsquerschnitt an einer anderen Stelle des Pfades der Substanz angeordnet, beispielsweise einem Abschnitt, in dem die Substanz zwischen dem Ventilkörper und der Innenwandung des Gehäuses hindurch tritt, so wäre die aus dem Ventil austretende Menge an Substanz sehr empfindlich von der genauen Stellung des ventilkörpers gegenüber dem Gehäuse abhängig.

Vorzugsweise ist der Strömungsquerschnitt in anderen Bereichen des Pfades der Substanz (anderen Bereichen als der Austrittsöffnung) größer als das 1,5-fache des Strömungsquerschnitts der Austrittsöffnung, bevorzugt größer als das 2-fache und besonders bevorzugt größer als das 4-fache des Strömungsquerschnitts der Austrittsöffnung.

Bevorzugt handelt es sich bei der Austrittsöffnung um eine Blende bzw. die Austrittsöffnung ist in einer Blende angeordnet. Auf diese Weise ist es möglich, den Querschnitt dieser Blende sehr genau zu bestimmen, wobei beispielsweise im Falle einer kreisförmigen Blende dieser Querschnitt durch eine genau ermittelbare Kreisfläche gegeben ist. Vorzugsweise ist diese Blende aus einem Material hergestellt, welches gegenüber den Einwirkungen von Wasserstoffperoxid resistent ist.

Vorzugsweise weist der Ventilkörper an seinem der Austrittsöffnung zugewandten Endabschnitt einen sich in Richtung der Austrittsöffnung verjüngenden Verschlusskörper auf. Dieser Verschlusskörper kann innerhalb des Gehäuses, das heißt genauer, innerhalb der Innenwandung des Gehäuses geführt werden und auf diese Weise in einer Stellung das Ventil schließen und in einer weiteren Stellung das Ventil öffnen. Durch diesen sich verjüngenden Abschnitt wird erreicht, dass bei einer vorgegebenen Öffnungsstellung des Ventils der Strömungsquerschnitt auf dem Pfad der Substanz vor der Austrittsöffnung, der durch den Außenumfang des Verschlusskörpers und den Innenumfang des Gehäuses bestimmt wird, größer ist als der Strömungsquerschnitt der Austrittsöffnung.

Bevorzugt ist in einem geöffneten Zustand des Dosierventils der Abstand zwischen der Außenkontur des Ventilkörpers und insbesondere der Außenkontur des Verschlusskörpers und der Innenoberfläche des Gehäuses größer als der Durchmesser der Austrittsöffnung. Bevorzugt beträgt das Verhältnis zwischen diesem Abstand und dem Durchmesser der Austrittsöffnung wenigstens 1,5, bevorzugt wenigstens 2 und besonders bevorzugt wenigstens 4.

Bei einer vorteilhaften Ausführungsform weist der sich verjüngende Verschlusskörper eine kegelstumpfförmige Außenkontur auf. Anstelle einer kegelstumpfförmigen Außenkontur wären jedoch auch andere sich in Richtung der Austrittsöffnung verjüngende Außenkonturen wie gekrümmte oder halbkugelförmige Außenkonturen denkbar.

Vorzugsweise ist der Außenumfang des Verschiusskörpers an einen Innenumfang des Gehäuses angepasst. Genauer gesagt ist der Außenumfang des Verschlusskörpers in dem Bereich, in dem sich der Verschlusskörper gegenüber dem Gehäuse oder der Innenwandung des Gehäuses bewegt, an die Kontur dieser Innenwandung angepasst. Auf diese Weise kann erreicht werden, dass das Ventil in seiner Schließstellung zuverlässig geschlossen wird und in seiner Öffnungsstellung einen Strömungsquerschnitt bildet, der größer ist als der durch die Austrittsöffnung gebildete Strömungsquerschnitt.

Insbesondere im Falle einer konischen Gestalt kann ein zuverlässiges Schließen und Öffnen des Ventils erreicht werden.

Vorzugsweise schließt sich oberhalb des Verschlusskörpers ein zylindrischer Bereich des Ventilkörpers an. Auf diese Weise wird ein gleichförmiger Strömungsquerschnitt oberhalb des Verschlusskörpers erreicht. Bei einer weiteren vorteilhaften Ausführungsform weist das Dosierventil eine Balgdichtung auf. Diese Balgdichtung, die besonders vorteilhaft aus Teflon hergestellt ist, erlaubt eine besonders vorteilhafte Führung des Ventilkörper innerhalb des Gehäuses.

Bei einer weiteren vorteilhaften Ausführungsform ist die Zuleitung in einer Mantelfläche des Dosierventils bzw. dessen Gehäuse angeordnet. Vorteilhaft läuft die Zuleitung nicht horizontal sondern ist schräg nach unten, das heißt, sie ist in Richtung der Austrittsöffnung des Dosierventils geneigt.

Bei einer weiteren vorteilhaften Ausführungsform ist das Dosierventil durch einen pneumatischen Antrieb betätigbar. Durch diesen pneumatischen Antrieb kann der Verschlusskörper gegenüber dem Gehäuse bewegt werden. Es wären jedoch auch andere Antriebe denkbar wie beispielsweise piezoelektrische Antriebe, magnetische Antriebe oder dergleichen denkbar.

Bei einer weiteren vorteilhaften Ausführungsform ist eine Federeinrichtung vorgesehen, die den Ventilkörper in eine bestimmte Stellung vorspannt. In dieser Stellung ist das Dosierventil bevorzugt geschlossen. Damit handelt es sich bei dem erfindungsgemäßen Dosierventil vorzugsweise um ein sogenanntes "normally closed"-Ventil.

Vorzugsweise schließt sich unterhalb der Blende ein Gehäusebereich mit gegenüber der Blende vergrößerten Querschnitt an. Daneben kann unterhalb der Blende auch ein Deckel mit einer sich ausgehend von der Blende erweiternden Ausnehmung vorgesehen sein.

Die vorliegende Erfindung ist weiterhin auf einen Verdampfer zum Sterilisieren von Behältnissen mittels einer Wasserstoffperoxid enthaltenden Substanz gerichtet, wobei dieser Verdampfer wenigstens ein Dosierventil der oben beschriebenen Art aufweist. Vorzugsweise weist der Verdampfer eine Heizeinrichtung zum Verdampfen des Wasserstoffperoxids auf und das Dosierventil ist derart angeordnet, dass das Wasserstoffperoxid zumindest auch in einer senkrecht zu einer Oberfläche dieser Heizeinrichtung stehenden Richtung auf die Heizeinrichtung ausgegeben wird. Eine Anordnung des Dosierventils, bei der die Substanz senkrecht nach unten austreten kann, ist besonders bevorzugt.

Weitere Vorteile und Ausführungsformen ergeben sich aus den beigefügten Zeichnungen:

Darin zeigen:
- Fig. 1: einen Verdampfer mit einem erfindungsgemäßen Dosierventil, wobei das Dosierventil in einer geschlossenen Stellung ist;
- Fig. 2: den Verdampfer mit Dosierventil aus Fig. 1, wobei das Ventil in einer geöffneten Stellung ist;
- Fig. 3: eine Ansicht einer Austrittsöffnung; und
- Fig. 4 und 5: eine Gesamtdarstellung eines erfindungsgemäßen Dosierventils.

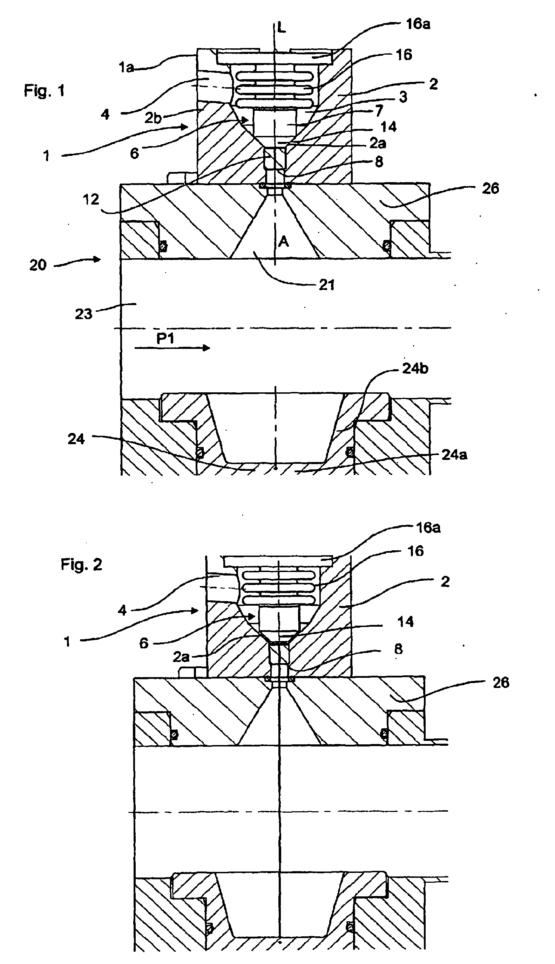

Fig. 1 zeigt eine teilweise Darstellung eines Verdampfers 20 mit einem erfindungsgemäßen Dosierventil 1. Dabei kann entlang einer Führungsstrecke 23 entlang des Pfeils P1 ein gasförmiges Medium wie beispielsweise erwärmte Luft durch den Verdampfer 20 geführt werden. Diese erwärmte Luft nimmt anschließend die von dem Dosierventil 1 auf eine Heizeinrichtung 24 ausgegebene und anschließend von dort verdampfte Wasserstoffperoxidlösung mit und führt diese in Richtung der zu sterilisierenden (nicht gezeigten) Behältnisse.

Die erfindungsgemäße Vorrichtung ist jedoch nicht auf das in Fig. 1 gezeigte Prinzip beschränkt, es ist auch möglich, dass die Luftzuführung nicht quer verläuft, sondern in einer Längsrichtung, d. h. parallel zu der Zuführungsrichtung des H₂O₂. Die Heizeinrichtung 24 ist derart gestaltet, dass nicht nur deren Bodenfläche 24a sondern auch deren Seitenflächen 24b beheizt werden. Dies ist insofern günstig, als in diesem Falle auch von der Mitte nach außen tretendes Wasserstoffperoxid noch zuverlässig verdampft werden kann. In dem oberen Bereich des Verdampfers ist ein Deckel 26 angeordnet und an diesem Deckel 26 wiederum das Dosierventil 1. Der Deckel weist eine sich von oben nach unten erweiternde Ausnehmung auf, in deren Zentrum die Austrittsrichtung der Substanz verläuft.

Das Bezugszeichen 2 bezieht sich auf ein Gehäuse des Dosierventils, wobei innerhalb dieses Gehäuses ein Aufnahmeraum 3 gebildet wird, in dem ein Ventilkörper 6 entlang der Richtung L verschiebbar ist. Die Richtungen L und A stimmen miteinander überein. Fig. 1 zeigt eine geschlossene Stellung des Dosierventils 1, das heißt eine Stellung, bei der ein Verschlusskörper 14 des Ventilkörpers 6 einen entsprechenden unteren Abschnitt 2a des Gehäuses 2 vollständig verschließt. Dieser Verschlusskörper 14 ist über einen zylindrischen Körper 7 an einer Balgdichtung 16 angeordnet. Das Bezugszeichen 2b bezieht sich auf eine Außenwandung bzw. Mantelfläche des Gehäuses 2, in der eine Zuleitung 4 vorgesehen ist, über die Wasserstoffperoxid dem Dosierventil 1 zugeführt wird. Wie aus Fig. 1 ersichtlich, verläuft diese Zuleitung 4 nicht horizontal sondern ist gegenüber der horizontalen Richtung leicht geneigt. Auf diese Weise wird eine Zuführung des Wasserstoffperoxids in das Dosierventil 1 erleichtert.

Das Bezugszeichen 8 bezieht sich auf eine (nicht genauer gezeigte) Austrittsöffnung, durch welche das Wasserstoffperoxid austreten und auf die Heizeinrichtung 24 gelangen kann. Diese Austrittsöffnung 8 ist hier in einem topfartigen Körper 12 angeordnet. Dieser topfartige Körper 12 ist vorzugsweise vollständig mit Wasserstoffperoxid gefüllt. Im geschlossenen Zustand des Ventils 1 tritt kein. Wasserstoffperoxid in den topfartigen Körper 12 und damit auch nicht durch die Austrittsöffnung 8 in den Verdampfer ein.

Fig. 2 zeigt das Dosierventil aus Fig. 1 in einer geöffneten Stellung. Man erkennt, dass hier der Verschlusskörper 14 geringfügig nach oben verschoben ist und damit der Weg für eine Zuführung von Wasserstoffperoxid zu der Austrittsöffnung 8 freigegeben wird. Ober die Zuleitung 4 wird Wasserstoffperoxid unter einem Druck eingeführt, der oberhalb des atmosphärischen Drucks liegt. Dadurch wird letztlich das Wasserstoffperoxid über den topfartigen Körper 12 und die Austrittsöffnung 8 in den Verdampfer eingeführt. Im geöffneten Zustand des Dosierventils fließt das H₂O₂ zwischen der Außenoberfläche des kegelstumpfartigen Verschlusskörpers 14 und der Innenwandung des Gehäuses hindurch. Der Strömungsquerschnitt in diesem Bereich ist jedoch größer als der Strömungsquerschnitt, der durch die Austrittsöffnung 8 gebildet wird.

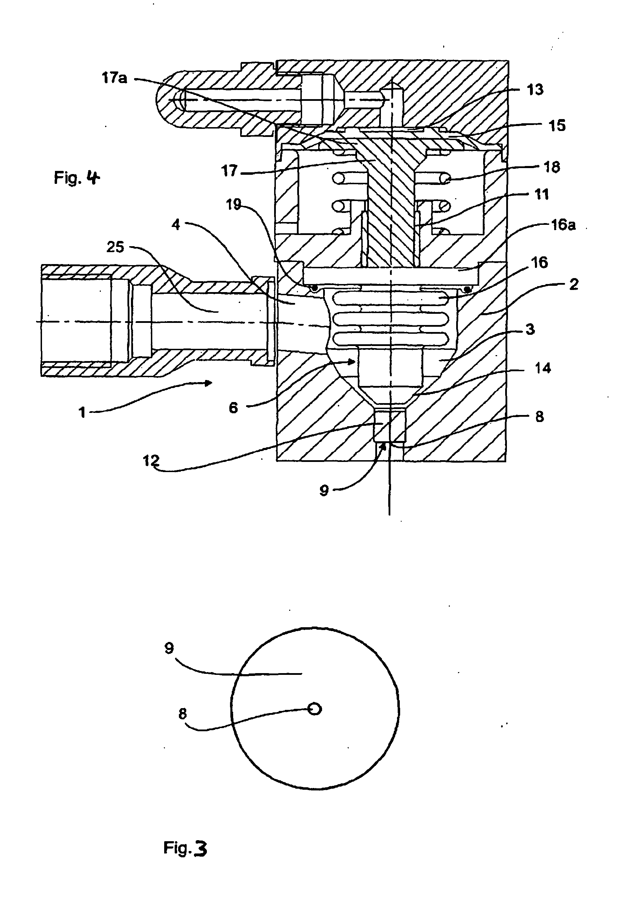

Fig. 3 zeigt eine Draufsicht auf eine erfindungsgemäße Austrittsöffnung 8. Bei dieser Ausführungsform ist diese Austrittsöffnung in einer Blende 9 angeordnet, die in Form einer Scheibe 9 ausgebildet sein kann. Diese Scheibe 9 ist aus einem Material hergestellt, welches resistent gegenüber den Wirkungen von Wasserstoffperoxid ist. Anstelle der in Fig. 3 gezeigten Ausführungsform könnte jedoch die Blende 9 auch in einem insgesamt einteilig ausgeführten topfartigen Gebilde vorgesehen sein. Der Durchmesser der kreisrunden Austrittsöffnung 8 liegt zwischen 0,05 mm und 0,4mm, bevorzugt zwischen 0,1mm und 0,3mm.

Fig. 4 zeigt eine Ausführungsform eines erfindungsgemäßen Dosierventils 1 in seiner Gesamtheit. Man erkennt, dass sich an die Zuleitung 4 von außen her eine Zuführeinrichtung 25 anschließt. Diese Zuführeinrichtung 25 könnte auch selbst gegenüber dem Gehäuse 2 in ähnlicher Weise geneigt sein, wie die Zuleitung 4. Bei der in Fig. 4 gezeigten Ausführungsform wird das Dosierventil 1 pneumatisch gesteuert. Dazu wird ein Luftraum 13 zum Schließen des Ventils mit Druckluft beaufschlagt. Durch die Beaufschlagung des Luftraums 13 mit Druckluft wird eine unterhalb des Luftraums liegende Membran 15 nach unten gedrückt. Diese Membran 15 wiederum drückt auf einen Deckelabschnitt 17a eines Stößels 17. Dieser Stößel 17 mündet mit seinem unteren (nicht gezeigten) Ende-in den Verschlusskörper 14.

Damit wird im Falle einer Druckbeaufschlagung der Verschlusskörper 14 nach unten bewegt und auf diese Weise das Dosierventil geschlossen. Falls das Dosierventil nicht mit Druckluft beaufschlagt ist, befindet es sich in der geöffneten Stellung. Zu diesem Zweck ist eine Federeinrichtung 18 vorgesehen, die gegenüber dem Gehäuse 2 bzw. einem Oberteil 16a der Dichtung 16 abgestützt ist. Das andere Ende dieser Federeinrichtung 18 wirkt von unten auf den Deckelabschnitt 17a des Stößels 17 und drückt damit den Stößel in seiner Gesamtheit nach oben, das heißt in Richtung der geöffneten Stellung. Die Konstruktion kann aber auch so abgewandelt werden, dass die Federeinrichtung den Stößel bevorzugt in Richtung einer geschlossenen Stellung beaufschlagt.

Das Bezugszeichen 11 bezieht sich auf eine Gleitlagerbuchse, die zur Führung des Stößels 17 gegenüber dem Gehäuse 2 vorgesehen ist. Das Bezugszeichen 19 bezieht sich auf eine Dichtungseinrichtung zum Abdichten der beiden in Fig. 4 gezeigten Gehäuseteile des Gehäuses 2.

In einer weiteren Ausführungsform wäre es möglich, nicht nur eine Austrittsöffnung 8 vorzusehen sondern innerhalb des topfartigen Gebildes einen Austrittskanal, der sich mit dem konstanten Querschnitt (der Austrittsöffnung) in der Längsrichtung L erstreckt.

Sämtliche in den Anmeldungsunterlagen offenbarten Merkmale werden als erfindungswesentlich beansprucht, sofern sie einzeln oder in Kombination gegenüber dem Stand der Technik neu sind.

Die Erfindung wird durch die Patentansprüche definiert.

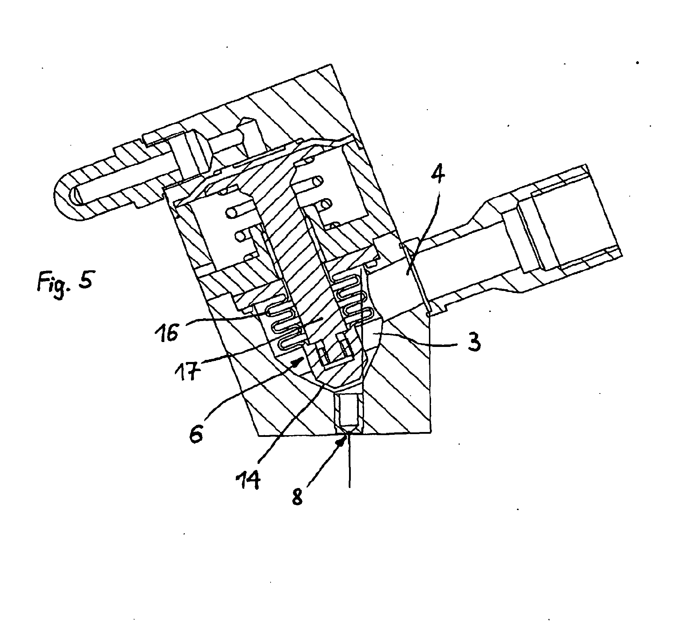

Die Ausführung nach Fig. 5 unterscheidet sich gegenüber der vorhergehend beschriebenen Variante nach Fig. 4 im Wesentlichen durch eine geneigte Anordnung des Stößels 17 in Bezug auf die Ebene der Austrittsöffnung des Dosierventils. Auch die Zuleitung 4 ist gegenüber dieser Ebene geneigt. Dementsprechend ist das Gehäuse in seiner Formgebung angepasst. Bei dieser Ausführung ergibt sich infolge der geneigten Bauform eine verbesserte Abführung von evtl. entstehenden Gasblasen im Wasserstoffperoxid.

## Patentansprüche

1. Dosierventil (1), insbesondere zum Dosieren einer Wasserstoffperoxid (H₂O₂) enthaltenden Substanz, mit einem Gehäuse (2), einer Zuleitung (4), um dem Dosierventil (1) die Substanz zuzuführen, mit einem innerhalb des Gehäuses (2) entlang einer geraden Richtung (L) zwischen wenigstens zwei Stellungen bewegbarem Ventilkörper (6) und mit einer in dem Dosierventil (1) angeordneten Austrittsöffnung (8) für die Substanz, wobei die Austrittsöffnung (8) einen vorgegebenen Strömungsquerschnitt aufweist und dieser Querschnitt in einer geöffneten Stellung des Dosierventils (1) den geringsten Strömungsquerschnitt des Dosierventils auf dem Pfad der Substanz durch das Dosierventil bildet.
**dadurch gekennzeichnet, dass**
der Ventilkörper (6) an seinem der Austrittsöffnung (8) zugewandten Endabschnitt einen sich in Richtung der Austrittsöffnung (8) verjüngenden Veschlusskörper (14) aufweist und der Aussenumfang des Verschlusskörpers (14) an einen Innenumfang des Gehäuses (2) angepasst ist.

2. Dosierventil (1) nach Anspruch 1,
**dadurch gekennzeichnet, dass**
die Austrittsöffnung (8) innerhalb einer Blende (9) angeordnet ist.

3. Dosierventil (1) nach Anspruch 1,
**dadurch gekennzeichnet, dass**
der sich verjüngende Verschluaskörper (14) eine kegelstumpfförmige Aussenkontur aufweist.

4. Dosierventil (1) nach wenigstens einem der vorangegangenen Ansprüche,
dadurch gekennzelchnet, dass
das Dosierventil (1) eine Balgdichlung (18) aufweist.

5. Dosierventil (1) nach wenigstens einem der vorangegangenen Ansprüche,
**dadurch gekennzeichnet, dass**
die Zuleiturig (4) in einer Mantelfläche (2b) des Dosierventils (1) angeordnet ist.

6. Dosierventil (1) nach wenigstens einem der vorangegangenen Ansprüche,
**dadurch gekennzeichnet, dass**
das Dosierventil (1) durch einen vorzugsweise pneumatischen oder elektromotorischen oder piezoelektrischen Antrieb betätigbar ist.

7. Dosierventil (1) nach wenigstens einem der vorangegangenen Ansprüche,
**dadurch gekennzeichnet, dass**
eine Federeinrichtung (18) vorgesehen ist, die den Ventilkörper (6) in eine Stellung vorspannt, in der das Dosierventil (1) bevorzugt geschlossen ist

8. Dosierventil (1) nach wenigstens einem der vorangegangenen Ansprüche 2 - 7,
**dadurch gekennzeichnet, dass**
sich unterhalb der Blende (9) ein Deckel mit einer sich ausgehend von der Blende (9) erweiternden Ausnehmung (21) anschließt.

9. Verdampfer (20) zum Sterilisieren von Behältnissen mittels einer Wasserstoffperoxid (H₂O₂) enthaltenden Substanz, mit einem Dosierventil (1) nach wenigstens einem der vorangegangenen Ansprüche.

10. Verdampfer (20) nach Anspruch 9,
**dadurch gekennzeichnet, dass**
der Verdampfer (20) eine Heizeinrichtung (24) zum Verdampfen des Wasserstoffperoxids aufweist und das Dosierventil (1) derart angeordnet ist, dass das Wasserstoffperoxid zumindest auch in einer senkrecht zu der Oberfläche der Heizeinrichtung (24) stehenden Richtung auf die Heizeinrichtung (24) ausgegeben wird.

## Claims

1. A metering valve (1), in particular for metering a substance containing hydrogen peroxide (H₂O₂), with a housing (2), a supply line (4) in order to supply the substance to the metering valve (1), with a valve member (6) movable between at least two positions along a straight direction (L) inside the housing (2), and with an outlet opening (8) situated in the metering valve (1) for the substance, wherein the outlet opening (8) has a pre-set flow cross-section and in an opened position of the metering valve (1) this cross-section forms the smallest flow cross-section of the metering valve on the path of the substance through the metering valve, **characterized in that** on its end portion facing the outlet opening (8) the valve member (6) has a closure member (14) tapering inwardly in the direction of the outlet opening (8), and the external periphery of the closure member (14) is adapted to an internal periphery of the housing (2).

2. A metering valve (1) according to claim 1, **characterized in that** the outlet opening (8) is situated inside an aperture (9).

3. A metering valve (1) according to claim 1, **characterized in that** the inwardly tapering closure member (14) has a frustoconical external contour.

4. A metering valve (1) according to at least one of the preceding claims, **characterized in that** the metering valve (1) has a bellows seal (16).

5. A metering valve (1) according to at least one of the preceding claims, **characterized in that** the supply line (4) is situated in an external area (2b) of the metering valve (1).

6. A metering valve (1) according to at least one of the preceding claims, **characterized in that** the metering valve (1) is capable of being actuated by a preferably pneumatic or piezoelectric drive or electric motor drive.

7. A metering valve (1) according to at least one of the preceding claims, **characterized in that** a spring device (18) is provided which pre-stresses the valve member (6) in a position in which the metering valve (1) is preferably closed.

8. A metering valve (1) according to at least one of the preceding claims 2 to 7, **characterized in that** a lid with a recess (21) increasing from the aperture (9) is attached below the aperture (9).

9. An evaporator (20) for the sterilization of containers by means of a substance containing hydrogen peroxide (H₂O₂), with a metering valve (1) according to at least one of the preceding claims.

10. An evaporator (20) according to claim 9, **characterized in that** the evaporator (20) has a heating device (24) for evaporating the hydrogen peroxide, and the metering valve (1) is arranged in such a way that the hydrogen peroxide is discharged onto the heating device (24) at least also in a direction at a right angle to the surface of the heating device (24).

## Revendications

1. Valve doseuse (1), destinée en particulier au dosage d'une substance contenant du peroxyde d'hydrogène (H₂O₂), avec un boîtier (2), un conduit d'amenée (4) destiné à alimenter en substance la valve doseuse (1), avec un corps de valve (6) déplaçable entre au moins deux positions le long d'une direction (L) droite à l'intérieur du boîtier (2) et avec une ouverture de sortie (8) pour la substance, ménagée dans ladite valve doseuse (1), l'ouverture de sortie (8) présentant une section d'écoulement définie et ladite section formant la section d'écoulement minimale de la valve doseuse sur le trajet de la substance au travers de la valve doseuse dans une position d'ouverture de la valve doseuse (1),
**caractérisée en ce qu'**,
à sa partie d'extrémité dirigée vers l'ouverture de sortie (8), le corps de valve (6) comporte un corps de fermeture (14) se rétrécissant dans la direction de l'ouverture de sortie (8), et **en ce que** la périphérie extérieure du corps de fermeture (14) est ajustée à une périphérie intérieure du boîtier (2).

2. Valve doseuse (1) selon la revendication 1,
**caractérisée en ce que**
l'ouverture de sortie (8) est disposée à l'intérieur d'un disque (9).

3. Valve doseuse (1) selon la revendication 1,
**caractérisée en ce que**
le corps de fermeture (14) qui se rétrécit présente un contour extérieur tronconique.

4. Valve doseuse (1) selon au moins une des revendications précédentes,
**caractérisée en ce que**
ladite valve doseuse (1) comporte un joint annelé (16).

5. Valve doseuse (1) selon au moins une des revendications précédentes,
**caractérisée en ce que**
le conduit d'amenée (4) est ménagé dans une surface périphérique (2b) de ladite valve doseuse (1).

6. Valve doseuse (1) selon au moins une dès revendications précédentes,
**caractérisée en ce que**
ladite valve doseuse (1) est actionnable par un entraînement préférentiellement pneumatique, électromoteur ou piézoélectrique.

7. Valve doseuse (1) selon au moins une des revendications précédentes,
**caractérisée en ce que**
un dispositif à ressort (18) est prévu, lequel contraint le corps de valve (6) dans une position où la valve doseuse (1) est préférentiellement fermée.

8. Valve doseuse (1) selon au moins une des revendications 2 à 7,
**caractérisée en ce que**
un couvercle avec un évidement (21) qui s'élargit à partir du disque (9) est contigu au disque (9) sous celui-ci.

9. Evaporateur (20) pour la stérilisation de récipients au moyen d'une substance contenant du peroxyde d'hydrogène (H₂O₂), avec une valve doseuse (1) selon au moins une des revendications précédentes.

10. Evaporateur (20) selon la revendication 9,
**caractérisé en ce que**
ledit évaporateur (20) comporte un dispositif de chauffage (24) pour l'évaporation du peroxyde d'hydrogène, et **en ce que** la valve doseuse (1) est disposée de telle manière que le peroxyde d'hydrogène soit délivré au dispositif de chauffage (24) au moins également dans une direction perpendiculaire à la surface du dispositif de chauffage (24).
